Europäisches Patentamt

European Patent Office

Office européen des brevets

⑩

(11) Publication number: **0 059 694**
**B1**

(12) **EUROPEAN PATENT SPECIFICATION**

(45) Date of publication of patent specification: **24.04.85**

(21) Application number: **82850011.6**

(22) Date of filing: **29.01.82**

(51) Int. Cl.⁴: **A 61 M 5/14,** A 61 D 7/00,
A 61 M 31/00

(54) **Drug administration device.**

(30) Priority: **26.02.81 SE 8101247**

(43) Date of publication of application:
**08.09.82 Bulletin 82/36**

(45) Publication of the grant of the patent:
**24.04.85 Bulletin 85/17**

(84) Designated Contracting States:
**AT BE CH DE FR GB IT LI LU NL SE**

(56) References cited:
**DE-A-1 942 027**
**DE-A-2 225 862**
**DE-A-2 459 321**
**FR-A-2 384 504**
**GB-A- 982 107**
**US-A-3 756 390**
**US-A-3 797 485**

(73) Proprietor: **Aktiebolaget Hässle**
**Kärragatan 5**
**S-431 83 Mölndal (SE)**

(72) Inventor: **Bogentoft, Conny Börje**
**Bäckvägen 12**
**S-430 50 Källered (SE)**
Inventor: **Boyes, Robert Nichol**
**6, Lancaster Road**
**St. Albans Herts, AL1 3JW (GB)**
Inventor: **Lambert, Hans Raymond**
**Askims kyrkväg 59A**
**S-436 00 Askim (SE)**
Inventor: **Sjögren, John Albert**
**Kullbäckstorpsvägen 40**
**S-435 00 Mölnlycke (SE)**

(74) Representative: **Wurm, Bengt Runio et al**
**Patent and Trade Mark Department AB ASTRA**
**S-151 85 Södertälje (SE)**

Courier Press, Leamington Spa, England.

## Description

Technical Field

The present invention is related to a device for administration of pharmaceuticals by a parenteral route. More specifically the invention is related to a device for use in an infusion system for release of an active substance to an infusion liquid. An object of the invention is to enable an improved control of the administration of a drug with infusion.

Background Art

Administration of drugs by infusion is a routine practice in medical care. The different problems encountered when administering drugs by infusion are described in many texts e.g. Sterile Dosage Forms by S. Turco and R. E. King, Lea and Febriger publishers 1979, (2nd edition), Philadelphia, USA. Normally, a drug in aqueous solution is mixed into an infusion solution and the mixture of solutions is administered via a catheter provided with flow control means and an infusion cannula. In order to obtain an even rate of administration of the drug the infusion flow rate must be carefully controlled. Control of the rate of drug infusion is often important for optimum therapeutic results. Control by means of catheter clamps and drip chambers is difficult to maintain. Variation of the flow rate occurs due to various circumstances e.g. clogging of the catheter, variation in blood pressure etc., and a variation of the drug administation rate follows, which often impairs the therapeutic result and which in certain cases may be fatal. In order to avoid administration of a large volume of liquid, it is often necessary to use a concentrated drug solution, which then must be infused slowly. The flow control problems are then even more difficult. An improved flow control can be obtained e.g. by using a mechanical pump such as a peristaltic pump, however, such pump renders the infusion system complicated, expensive and dependent on supply, of energy.

U.S. Patent No. 3 941 126 discloses an apparatus for administration of multiple medications, wherein an infusion solution from a common source is divided and led into several containers for drug solutions. The drug solutions drawn from said containers are then combined before infusion. While such device may overcome certain problems of incompatibility between different drugs, the device does not overcome problems connected with the drug administration rate, and further, it requires the drugs to be filled into the device in liquid form.

Swiss Patent No. 497 181 describes an infusion device having a drip-counting device provided with breakable closures at the inlet and outlet thereof and containing a liquid or solid additional material to be mixed or dissolved into the infusion liquid. The device is operated by first breaking the closure at the inlet of the drip-counting device and introducing therein part of the infusion liquid which mixes with or dissolves the additional material, whereupon said liquid is returned at least in part to the infusion container. This operation is repeated to complete mixing or dissolution of the additional material. Finally the closure at the outlet is broken to obtain infusion. The disclosure does not suggest a solution to the problem of controlling the drug administation rate on infusion, and further, release of a drug to a flow of liquid is not proposed.

Disclosure of Invention

The present invention is related to a drug administration device for releasing a drug into a flow of liquid during administration by a parenteral route to a patient, said device comprising a cell having an inlet and an outlet for the liquid so as to permit the liquid to flow through the cell, the cell containing a solid drug-releasing unit positioned to contact the liquid flowing through the cell in use, the drug-releasing unit being adapted to release the drug at a controlled rate into the liquid.

The term "drug" used herein encompasses agents having a medical effect, e.g. a therapeutic, alleviating or prophylactic effect on a patient but also agents administered for some other purposes, e.g. for diagnosis. The term "a patient" may be interpreted in a broad sense to include a human or animal host made subject to treatment.

The device of the invention provides a substantial improvement over previously used infusion systems, especially in relation to the problems referred to above. Thus, the rate of drug administration can be made less dependent on the infusion flow rate. Problems of instability in solution or incompatibility with the infusion solution will also be overcome.

The cell or compartment which contains the solid drug-releasing unit may be designed in several different ways. It may simply be a cylindrical container having an inlet opening and an outlet opening for the infusion liquid flow at opposite ends, or it may have some other shape ensuring contact between the flow of infusion liquid and the drug unit. The inlet opening may be provided with a drip-delivering means. Normally the device of the invention will be disposable after use, however, the device may also be rechargeable for repeated use if desired. A disposable device is preferably sealed and contains the drug delivering unit. When a device for repeated use or a device in which the drug delivering unit is to be inserted by the user is desired, the cell should be closable by means of a screw lid, a snap closure or the like. A sealed disposable device containing the drug delivering unit is preferred from hygienic and practical points of view and also with regard to safety in identification of the drug. A device of this type may also increase the safety for personnel handling dangerous or allergenic drugs. Filter means as well as flow control means may be included in the device.

The solid drug-releasing unit employed should be designed to have a controlled release profile suitable for the drug employed and the intended therapeutic treatment. Examples of drug-releasing units can be found in the literature e.g. Sustained and Controlled Release Drug Delivery Systems, Marcel Dekker, New York, 1978. The drug unit may i.e. comprise granules or one or more solid bodies such as tablets. The granules or tablets shall contain the drug, normally in conjunction with a carrier. Any carrier for a drug should be a pharmaceutically acceptable carrier in parenteral administration. The granules or tablets can be similar to granules or tablets for oral administration. Among granules are encompassed also micro-encapsulated compositions wherein the encapsulated drug may be in solid or liquid form.

According to the invention the solid drug-releasing unit has a controlled release profile e.g. giving delayed or sustained release of the drug. Numerous types of preparations giving controlled release are known in the art. Thus, to give a such release profile the unit may comprise granules or one or more tablets having a coating through which the drug will penetrate slowly. The tablets or granules may also have a matrix through which the drug will leach out, as known in the art of oral sustained-release preparations e.g. Durules®. The matrix of the drug-releasing unit may also consist of an ion exchange matrix which will give the drug a pH-dependent release. To obtain a certain release profile a tablet may have one or more bores all through or partly through the same.

The drug release characteristics of the device of the invention depend both on the release characteristics of the drug-releasing unit and on the design of cell in which it is to be held. By reducing the cell volume it is possible to obtain a device in which the released amount of drug per hour is very little dependent on the flow rate within wide limits. Thus, for such purposes a device in which the cell is designed to contain less than 10 ml of liquid in addition to the drug unit is preferred at flow rates normally used. Differently expressed, said cell should be designed to contain a volume of liquid less than 20% of the minimum volume of liquid that is to pass therethrough in one hour. By combining a cell having such small capacity with a drug unit giving sustained release, as referred to above, the drug administration rate can be made substantially independent of a flow rate varying within normal limits in infusion.

In a further embodiment, the drug-releasing unit is a coating of a drug composition on an inside wall of the cell in which it is contained.

In still a further embodiment the drug-releasing unit may consist of a separate container inside the device. This container has one or more holes through which the drug is released and through which the infusion solution is let into the container. Alternatively the infusion solution is let into such container through a membrane. A drug in liquid form may be released from such a container through a capillary.

The device of the invention should have the ability of providing a sterile infusion solution to the patient. Thus, the device is preferably provided sterilized e.g. with ethylene oxide, or by sterilization in an autoclave or with β- or γ-radiation. The drug unit may be sterilized in a similar manner or prepared in an aseptic manner. As an alternative or complement to sterilization, a sterilizing filter for the liquid, having a mesh size less than 0.2 μm, may be applied after the device. Further, the device may be provided in a hygienic package.

The device is suitably connectable in a simple manner e.g. by use of quick couplings. Thereby, the device may be made connectable to infusion systems in use at present as well as to new or specially adapted infusion systems. Couplings may also enable connection of two or more devices in series.

Preferably, the liquid volume in parts of the infusion system between the device and the patient should be small.

The drug administration device of the invention is useful for many different drugs. The device is of special interest for certain drug compounds of which should be mentioned compounds causing risks to the patient in excess dosages e.g. quinidine, digoxin, lidocaine, glucagon and other hormones and dexamethasone. Other compounds of this type could be cardiac glucosides, steriods, theophylline, isosorbide dinitrate, nitroglycerine; antineoplastic drugs e.g. methotrexate, nitrogen mustard and cyclophosphamide; L-hyoscyamine; protein drugs e.g. insulin; sedatives and anaesthetics such as different barbiturates; analgesics such as morphine; tranquilizers such as chloropromazine; cardiovascular agents not mentioned above e.g. dopamine; other agents like gentamicin sulphate; and agents for prophylactic use against deep vein thrombosis like heparin. Derivatives or salts of compounds mentioned may also be of interest.

In use, the device of the invention may be mounted vertically with the inlet for the liquid at the top end. This will be the case for a device having a drip-delivering inlet tube. However, the device may also be adapted to have the inlet at the lower end or it may be adapted to be held in any position, e.g. when it is to be attached to the body of the patient.

The rate of parenteral administration of a liquid may be varied between wide limits depending on the therapeutic requirements i.a. Typical flow rates for infusion are 100—300 ml/h.

The invention is further illustrated schematically by the attached drawings, of which

Fig. 1 shows a device according to one embodiment of the invention connected to a container for an infusion liquid,

Fig. 2 shows such a device according to the invention as a part of a complete infusion system, and

Fig. 3 to Fig. 10 show devices according to different embodiments of the invention, said devices being shown with the cell of each shown in section in Figs. 3, 4 and 6 to 10, whereby Figs. 4 and 5 show the same device viewed from two directions in 90° angle to each other.

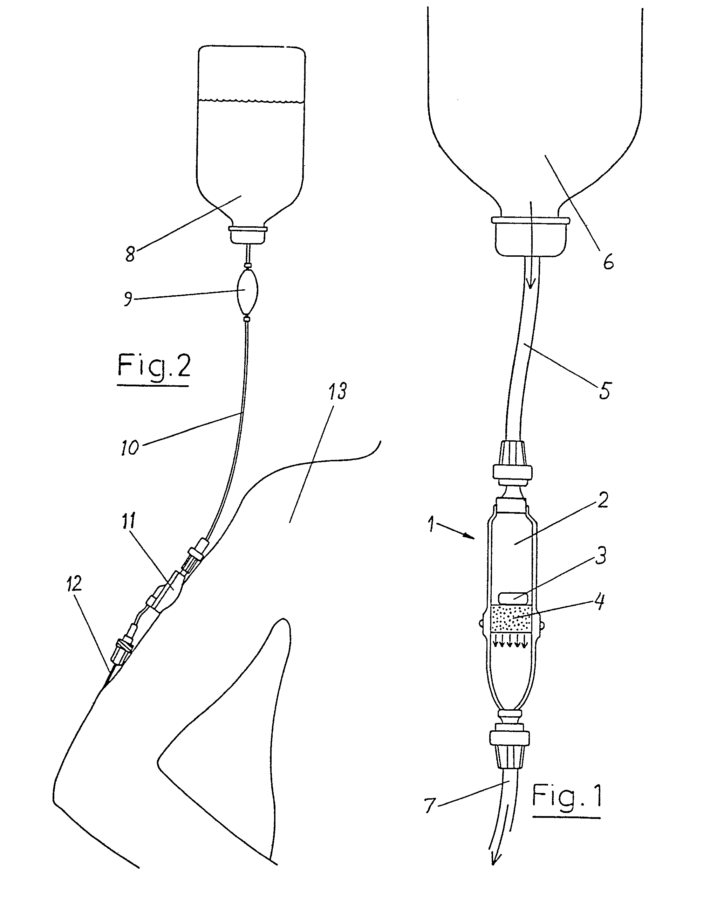

In Fig. 1, 1 denotes a drug administration device comprising a cell or compartment 2 wherein a solid drug release unit 3 in the form of a tablet is contained resting on a filter 4. The cell is connected at its upper end via an inlet duct 5 to a container 6 for an infusion liquid. An outlet duct 7 is connectable to an infusion catheter.

In Fig. 2 an infusion system comprising a container 8 for an infusion liquid, a drug administration device 9, an infusion catheter 10, a filter device 11 and an infusion cannula 12, is shown applied to a patient 13.

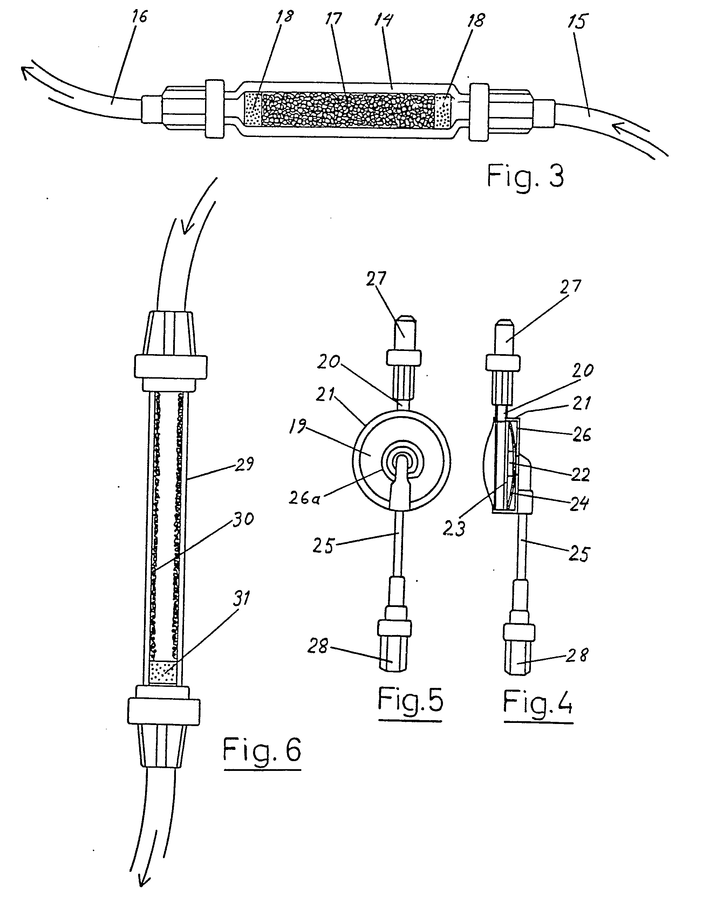

In Fig. 3 a cylindrical cell 14 connected to inlet and outlet ducts 15 and 16, respectively, arranged coaxially thereto, is filled with drug granules 17. Filters 18 prevent granules and other particles from leaving the cell.

In Figs. 4 and 5 a cell 19 is shown, having an outer appearance rather similar to certain sterile filter devices in current use, such as the filter device 11 of Fig. 2, said cell 19 being shaped like a low wide cylinder having an inlet duct 20 extending through its cylinder wall 21. A drug unit 22 is held between a perforated partition 23 and a filter 24. An outlet duct 25 is connected at the centre of the circular end wall 26 of the cell 19. A spiral edge 26a on the inside of wall 26 permits passage of liquid between said wall and the filter 24. Couplings 27 and 28 at the inlet duct 20 and the outlet duct 25 make the device easily connectable to other parts of an infusion system.

In Fig. 6 an elongated cylindrical container 29 has a coating 30 of a drug-releasing composition on its inner wall. A filter 31 near the outlet end prevents particles from leaving the device.

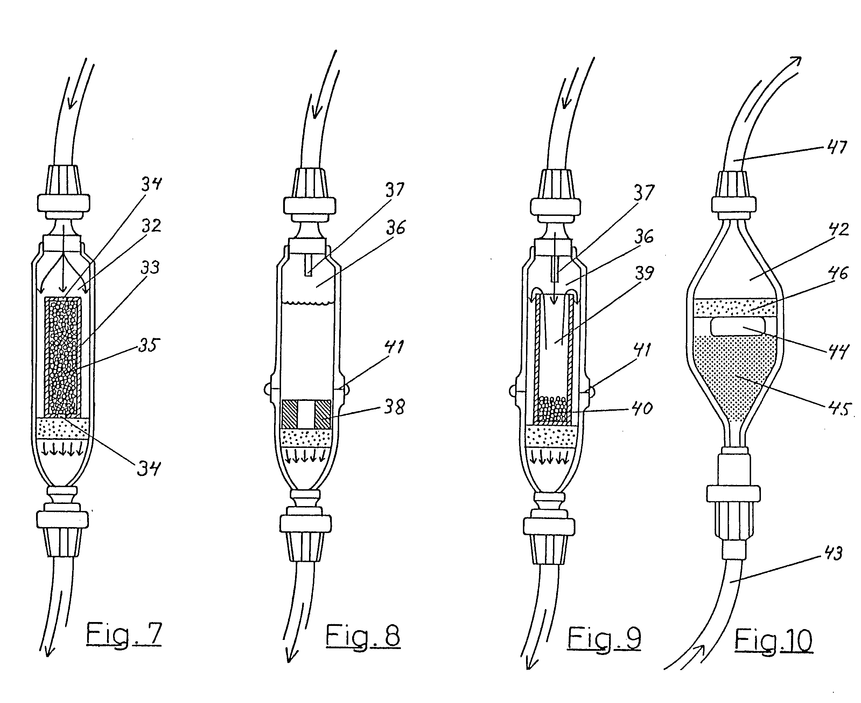

In Fig. 7 a cylindrical cell 32 contains a drug release unit comprising a container 33 having narrow openings 34 permitting contact between the infusion liquid and an easily soluble drug preparation 35 inside said container.

In each of Figs. 8 and 9 a cylindrical cell 36 into which a drip-delivering inlet tube 37 extends, contains as drug release unit, a hollow tablet body 38 or a tiny beaker 39 containing granules 40, respectively. The cell 36 may be disassembled at a joint 41 for insertion or exchange of the drug unit.

In Fig. 10 a cell 42 having circular cross section has an inlet duct 43 connected to the lower end thereof, in which cell 42 a solid drug release unit 44 is resting on glass beads 45. A filter 46 separates the unit 44 from the outlet duct 47 at the upper end of the cell 42.

In the drawings arrows indicate the intended flow direction. The devices of Figs. 3 to 7 may be used in any position while the devices of Figs. 8 to 10 should be used in the vertical position shown.

## Examples

The following examples further illustrate the invention and are not to be construed in a limiting sense.

## Example 1

An experimental set was constructed to illustrate some drug release characteristics of the invention. A drug release cell was designed as a cell shown in Fig. 9. The cell was mainly made of acrylic plastic. As a filter a fine mesh woven fibre was used. The cell contained a drug unit, supported by a solution distributor. The inside volume of the cell without the drug unit was approximately 35 ml. The drug cell was coupled via a plastic tube to a plastic bag containing an aqueous infusion solution with 5% by weight of $NaHCO_3$.

In order to determine the amount of drug released from the device, the outlet of the drug release cell was connected via a tube to a flow cell in an ultraviolet spectrophotometer. The solution was made flow through the drug release cell and the UV-spectrophotometer was used to detect the concentration of the drug in the infusion solution which would reach a patient.

A peristaltic pump was used to control the flow of infusion solution. The flow rate of the pump was variable within 18—800 ml/h. A pump is generally not needed in clinical practice and is only used for the purpose of controlling the flow rate of the infusion solution to obtain standardized experimental conditions.

A drug unit, essentially a matrix tablet, containing 250 mg of quinidine bisulphate, embedded in a matrix of polyvinylchloride and polyvinylacetate was enclosed in the drug cell. The flow rate was set to 90 ml/h and the quinidine absorption was followed by the UV-photometer for two hours. The concentration of the quinidine was calculated and by multiplying with the known flow rate of the infusion solution it was possible to calculate the release rate from the complete device. The cumulated amounts of drug released from the drug unit, including the amount remaining in the device were as shown in Table 1.

### TABLE 1

| Time, h | Released amount from drug unit, mg |
|---------|-----------------------------------|
| 0.5 | 62 |
| 1.0 | 94 |
| 2.0 | 135 |
| 4.0 | 181 |

**0 059 694**

The drug unit and the drug cell formed the complete device. The cumulated amounts of drug released from the device, as calculated from the same primary data as above, are shown in Table 2.

TABLE 2

| Time, h | Released amount from device, mg |
|---------|--------------------------------|
| 0.5 | 34 |
| 1.0 | 67 |
| 2.0 | 119 |
| 4.0 | 164 |

The design of the drug cell as well as the volume of tubing after the cell governs the amount of drug which eventually reaches the patient within a certain time.

Example 2

In an experimental set as described in Example 1 a device having a cell volume and volume of tubing of about 4 ml was studied. The release characteristics of this device is shown in Table 3. Except for the drug cell, the drug unit and the experimental set was the same as in Example 1.

TABLE 3

Released amoung of quinidine bisulphate (mg) from a device at different liquid flow rates

| Time, h | 55 ml/h | 90 ml/h | 180 ml/h |
|---------|---------|---------|----------|
| 1 | 86 | 89 | 90 |
| 2 | 128 | 130 | 131 |

Example 3

50 g of spherical beads of drug containing approximately 20% quinidine bisulphate were coated by spraying for 33 min. with ethylcellulose in a fluidized bed equipped with a spraying system according to known technology. The rate of ethylcellulose application was approximately 0.15 g/min. 1 g of these coated and dried beads was put into a cell having a volume of about 4 ml, to give the complete device. The complete set was similar to those used in Examples 1 and 2. Distilled water was pumped through the device at a rate of 117 ml/h. The release rate of quinidine bisulphate from the device was followed for 30 hours. The results are shown in Table 4.

TABLE 4

| Time, h | Release rate, mg/h |
|---------|---------------------|
| 1 | 1.5 |
| 5 | 4.5 |
| 10 | 3.8 |
| 15 | 3.5 |
| 20 | 3.5 |
| 25 | 3.5 |
| 30 | 3.5 |

5

**0 059 694**

As shown in Table 4, the release rate of quinidine bisulphate was constant for this particular device between 15 and 30 hrs. When the flow rate of the infusion solution was varied between 29 and 830 ml/h during this time it was found that the release rate was almost independent of the flow rate of the infusion solution as shown in Table 5.

TABLE 5

| Flow rate, ml/h | Released amount of quinidine bisulphate, mg/h |
|---|---|
| 29 | 3.22 |
| 60 | 3.43 |
| 117 | 3.52 |
| 120 | 3.58 |
| 428 | 3.58 |
| 830 | 3.35 |

Example 4

Granules were coated by spraying for 36 min in the same way as in Example 3, but the rate of application was changed to approximately 0.13 g/min. 1 g thereof (containing approximately 200 mg quinidine bisulphate) was put in the same set as Example 3. The release rate from device was followed for 45 hours. The results are given in Table 6.

TABLE 6

| Time, h | Release rate, mg/h |
|---|---|
| 1 | 11.5 |
| 5 | 5.1 |
| 10 | 3.8 |
| 20 | 3.5 |
| 30 | 3.5 |
| 45 | 3.2 |

Example 5

Small spherical beads containing approximately 33% isosorbidedinitrate (ISDN) were coated with a water-insoluble polymeric coating. 1 g of these coated granules were placed in a cell having a volume of about 4 ml of make a device of the invention. The device was connected to a solution container with distilled water and a pump. The water was pumped through the cell at approximately 10 ml/h. The amount of ISDN released from the device was followed for three hours. Samples were taken every hour and analyzed with a polarigraphic method for amount of drug released. The results are summarized in Table 7.

TABLE 7

| Time, h | Released amount of ISDN, mg/h |
|---|---|
| 1 | 1.5 |
| 2 | 1.5 |
| 3 | 1.5 |

6

# 0 059 694

Best Mode of Carrying Out the Invention

The best mode of carrying out the invention contemplated by the inventors is providing a device as shown in Fig. 4 wherein the cell contains granules coated with an insoluble membrane and giving constant release of a drug, such as lidocaine in an amount of 2.0 g in 6.7 g of granules releasing about 2 mg/min of lidocaine, whereby said cell has a capacity of about 14 ml of infusion liquid in addition to the granules, and whereby the filter of the device has a mesh size of 0.2 µm.

In the claims appended hereto reference numerals given in brackets refer to the drawings and are intended merely for illustration of the claims. The use of reference numerals is not to be construed as limiting the scope of protection afforded by the claims.

## Claims

1. A drug administration device for releasing a drug into a flow of liquid during administration by a parenteral route to a patient, comprising a cell (2, 14, 19, 29, 32, 36, 42) having an inlet and an outlet for the liquid so as to permit the liquid to flow through the cell, the cell containing a solid drug-releasing unit (3, 17, 22, 30, 33, 38, 39, 44) positioned to contact the liquid flowing through the cell in use, the drug-releasing unit being adapted to release the drug at a controlled rate into the liquid.

2. A device according to claim 1, wherein the cell is non-rechargeable and disposable after use.

3. A device according to claim 1 or 2, wherein the solid drug-releasing unit comprises granules or one or more tablets.

4. A device according to claim 3 wherein the granules or tablets have a coating through which the drug will penetrate slowly.

5. A device according to claim 3 wherein the granules or tablets have a matrix through which the drug will leach out.

6. A device according to one or more of claims 1 to 5 giving a drug administration rate substantially independent of a flow rate of the infusion liquid varying between normal limits in infusion, wherein the cell is designed to contain a volume of liquid less than 20% of the minimum volume of liquid to pass therethrough in one hour and that the drug-releasing unit is a unit giving sustained drug release.

## Revendications

1. Un dispositif d'administration de produits pharmaceutiques pour la libération d'un produit pharmaceutique dans un courant de liquide au cours de son administration à un malade par voie parentérale, comprenant une cellule (2, 14, 19, 29, 32, 36, 42) avec une entrée et une sortie pour le liquide de manière à permettre l'écoulement du liquide à travers la cellule, la cellule contenant une unité solide de libération de produit pharmaceutique (3, 17, 22, 30, 33, 38, 39, 44) placée au contact du liquide s'écoulant à travers la cellule en utilisation, l'unité de libération de produit pharmaceutique étant destinée à libérer à vitesse réglée le produit pharmaceutique dans le liquide.

2. Un dispositif selon la revendication 1, caractérisé en ce que la cellule est non rechargeable et jetable après usage.

3. Un dispositif selon la revendication 1 ou 2, caractérisé en ce que l'unité solide de libération de produit pharmaceutique contient des granules ou un ou plusieurs comprimés.

4. Un dispositif selon la revendication 3, caractérisé en ce que les granules ou les comprimés comportent un enrobage à travers lequel le produit pharmaceutique passe lentement.

5. Un dispositif selon la revendication 3, caractérisé en ce que les granules ou les comprimés comportent une matrice à travers laquelle le produit pharmaceutique est extrait par lixiviation.

6. Un dispositif selon une ou plusieurs des revendications 1 à 5, assurant une vitesse d'administration de produit pharmaceutique pratiquement indépendante du débit du liquide de perfusion variant dans les limites normales de la perfusion, caractérisé en ce que la cellule est conçue de manière à contenir un volume de liquide inférieur à 20% du volume de liquide minimal qui doit la traverser en une heure et que l'unité de libération de produit pharmaceutique est une unité assurant une libération prolongée de produit pharmaceutique.

## Patentansprüche

1. Vorrichtung zum Verabreichen von Arzneien, bei welcher eine Arznei in einen Flüssigkeitsstrom während der Verabreichung an einen Patienten auf parenteralem Weg abgegeben wird, gekennzeichnet durch eine Zelle (2, 14, 19, 29, 32, 36, 42) mit einem Eingang und einem Ausgang für die Flüssigkeit, wodurch ein Durchströmen der Flüssigkeit durch die Zelle ermöglicht wird, und wobei die Zelle eine feste Arzneiabgabeeinheit (3, 17, 22, 30, 33, 38, 39, 44) enthält, welche so angeordnet ist, daß sie während der Verwendung in Kontakt mit der die Zelle durchströmenden Flüssigkeit ist, und wobei die Arzneiabgabeeinheit so beschaffen ist, daß sie die Arznei mit einer gesteuerten Geschwindigkeit in die Flüssigkeit abgibt.

2. Vorrichtung nach Anspruch 1, dadurch gekennzeichnet, daß die Zelle nach ihrer Verwendung nicht wieder füllbar und wegwerfbar ist.

3. Vorrichtung nach Anspruch 1 oder 2, dadurch gekennzeichnet, daß die feste Arzneiabgabeeinheit Granalien oder eine oder mehrere Tabletten umfaßt.

4. Vorrichtung nach Anspruch 3, dadurch gekennzeichnet, daß die Granalien oder Tabletten einen Überzug aufweisen, durch welchen die Arznei langsam hindurchdringt.

5. Vorrichtung nach Anspruch 3, dadurch gekennzeichnet, daß die Granalien oder Tabletten eine Matrix aufweisen, durch welche die Arznei nach außen durchsickert.

6. Vorrichtung nach einem oder mehreren der Ansprüche 1 bis 5 mit einer Arzneiabgabegeschwindigkeit, die im wesentlichen von der zwischen für eine Infusion üblichen Grenzen variierenden Strömungsgeschwindigkeit der Infusionsflüssigkeit unabhängig ist, dadurch gekennzeichnet, daß die Zelle so beschaffen ist, daß sie ein Flüssigkeitsvolumen von weniger als 20% des während einer Stunde hindurchströmenden Flüssigkeitsmindestvolumens enthält und daß die Arzneiabgabeeinheit eine Einheit mit andauernder Arzneiabgabe ist.

Fig.2

Fig.1

Fig. 3

Fig. 5    Fig. 4

Fig. 6

2

Fig.7  Fig.8  Fig.9  Fig.10

0 059 694